# EUROPEAN PATENT APPLICATION

(11) **EP 4 775 632 A1**
(43) Date of publication of application: **15.07.2026**
(21) Application number: 24862755.6
(22) Date of filing: 02.09.2024
(51) Int. Cl.: C08L 25/04, C07H 9/04, C08K 5/06, C08L 33/04, C08L 69/00

(54) **RESIN COMPOSITION EXCELLENT IN TRANSPARENCY AND MOLDED ARTICLE THEREOF**

(30) Priority: 06.09.2023 JP 2023144656
(71) Applicant: Hemicellulose Ltd., Kawasaki-shi, Kangawa 212-0032 (JP)
(72) Inventor: ISHIKAWA Kenya, Kawasaki-shi, Kanagawa 212-0032 (JP); ANDO Keita, Kawasaki-shi, Kanagawa 212-0032 (JP); KAMO Yoko, Kawasaki-shi, Kanagawa 212-0032 (JP); MAEDA Yuhei, Kawasaki-shi, Kanagawa 212-0032 (JP); TSUTSUMI Yoshitaka, Kawasaki-shi, Kanagawa 212-0032 (JP)
(74) Representative: Fabry, Bernd
(86) International application number: PCT/JP2024/031454
(87) International publication number: WO 2025/053103

(57) **Abstract**

To provide a resin composition that solves the problem of reducing the usage ratio of raw materials derived from petroleum resources while maintaining the inherent transparency of a resin and the characteristics of heat resistance, mechanical properties, and moldability. The present embodiment is a resin composition having excellent transparency. The resin composition includes a base resin containing at least one of a styrenic resin, a polycarbonate resin, and an acrylic resin, and an acetalized compound in which at least a part of hydroxyl groups of a monosaccharide or an oligosaccharide has reacted with aldehydes. The base resin has a content ratio in the resin composition of 70% by mass or more and 97% by mass or less, and the acetalized compound has a molecular weight of 200 or more and 1,000 or less.

## Description

### [TECHNICAL FIELD]

The present invention relates to a resin composition excellent in transparency and to a molded article molded from the resin composition.

### [BACKGROUND ART]

With respect to conventional biomass-derived technologies for transparent resins, supply by a mass balance method via biomass naphtha has begun. This technology is capable of production using existing facilities and has advantages such as quality equivalent to that derived from fossil resources; however, there are concerns regarding the supply aspect due to difficulties in increasing the biomass blending ratio. Therefore, it has also been proposed, as one method for reducing environmental load, to provide a biomass-derived resin composition by mixing a petroleum-derived resin with a biomass-derived resin as an additive while maintaining mechanical properties and moldability.

As one example thereof, Patent Document 1 discloses a styrene-based resin containing polylactic acid which has low environmental load and excellent impact resistance. Patent Document 2 discloses a resin composition containing a polysaccharide derivative excellent in heat resistance, hardness, and the like, and Patent Document 3 discloses a styrene-based resin containing a cellulose-based polysaccharide excellent in light transmittance, heat resistance, and appearance. Patent Document 4 discloses a styrene-based resin containing a biopolyester excellent in transparency and heat resistance, and Patent Document 5 discloses a styrene-based resin containing a biomass-derived plasticizer such as vegetable oil, which is excellent in reducing environmental load and reducing mold contamination.

Further, Patent Document 6 discloses a biopolycarbonate obtained by kneading polylactic acid, which is a biomass-derived resin, with a general-purpose polycarbonate. Patent Document 7 discloses a biopolycarbonate in which conventional bisphenol A is replaced with a sugar-derived isosorbide-modified compound. Patent Document 8 discloses a technique for producing methacrylic acid and/or an ester thereof using microorganisms.

However, JP2015-86251A and JP2020-132790A contain an incompatible resin blended with a styrene-based resin, and thus it is difficult to maintain high transparency. JP2021-165378A contains a large amount of powdered cellulose, and thus high transparency cannot be obtained. In JP2022-134223A, degradation of the polyester is unavoidable, and high transparency also cannot be obtained. JP2023-25620A contains rubbery particles, and therefore it cannot be expected to obtain a styrene-based resin having high transparency. In addition, since JP2007-191577A contains an incompatible resin, and JP2012-92235A introduces a biomass component into a polycarbonate resin and additionally introduces wood flour, high transparency cannot be obtained in each case. WO2014/038216A requires that an acrylic resin raw material component derived from microorganisms be obtained at high purity, and therefore the technique is highly difficult and costly.

### [SUMMARY OF THE INVENTION]

### [PROBLEM TO BE SOLVED BY THE INVENTION]

Accordingly, the present invention provides a resin composition that solves the problem of reducing the usage ratio of raw materials derived from petroleum resources while maintaining the inherent transparency of a resin and the characteristics of heat resistance, mechanical properties, and moldability.

### [MEANS FOR SOLVING THE PROBLEM]

The present embodiment is a resin composition having excellent transparency. The resin composition includes a base resin containing at least one of a styrenic resin, a polycarbonate resin, and an acrylic resin, and an acetalized compound in which at least a part of hydroxyl groups of a monosaccharide or an oligosaccharide has reacted with aldehydes. The base resin has a content ratio in the resin composition of 70% by mass or more and 97% by mass or less, and the acetalized compound has a molecular weight of 200 or more and 1,000 or less.

The base resin preferably has a content ratio in the resin composition of 85% by mass or more and 95% by mass or less. The aldehydes are preferably derived from biomass. The aldehydes are at least one of anisaldehyde, cinnamaldehyde, furfural, and glyoxylic acid. Furthermore, the aldehydes are preferably cinnamaldehyde. A molded article of the present embodiment uses these resin compositions having excellent transparency.

The resin composition of the present invention can maintain the characteristics of transparency, heat resistance, mechanical properties, and moldability while reducing the usage ratio of raw materials derived from petroleum resources.

### [DETAILED DESCRIPTION OF THE INVENTION]

### <<Configuration of Resin Composition>>

The resin composition of the embodiment includes (a) a base resin containing at least one of a styrenic resin, a polycarbonate resin, and an acrylic resin, and (b) an acetalized compound in which at least a part of hydroxyl groups of a monosaccharide or an oligosaccharide has reacted with aldehydes. The content ratio of the (a) base resin in the resin composition is 70% by mass or more and 97% by mass or less. When the content ratio is less than 70% by mass, high transparency cannot be obtained, and when the content ratio exceeds 97% by mass, a high biomass component ratio cannot be obtained. Furthermore, the content ratio of the (a) base resin in the resin composition is preferably 80% by mass or more and 95% by mass or less, and more preferably 85% by mass or more and 90% by mass or less.

By blending the acetalized compound into the (a) base resin, it is possible to obtain a resin composition having excellent heat resistance, mechanical properties, and moldability while maintaining transparency. The principle thereof is still unclear; however, the (a) base resin is a so-called amorphous polymer and has characteristics of inhibiting crystallization due to large side chains and a main chain that bends significantly, thereby exhibiting high transparency.

In addition, since the acetalized compound of the present embodiment forms a plurality of cyclic acetal structures on the outer side around the raw material sugar, the acetalized compound is a compound that is relatively bulky but has low rigidity. Due to this characteristic, the acetalized compound is likely to approach the polymer chains of the (a) base resin and exhibits a characteristic of further disturbing the structure, and therefore it is considered that even when blended in a large amount, the resin composition maintains transparency while exhibiting high heat resistance, mechanical properties, and moldability.

The resin composition of the present embodiment can be a resin composition having a total light transmittance of 80% or more and 93% or less by selecting appropriate molding conditions and composition. A preferable range of the total light transmittance is 85% or more, a more preferable range is 87% or more, and an even more preferable range is 88% or more. When the total light transmittance is less than 80%, the target transparency cannot be achieved, and a total light transmittance exceeding 93% is a value that is difficult to achieve because reflected light is inevitable in view of the measurement principle. The total light transmittance is a value measured in accordance with JIS K 7375:2008.

The resin composition can be a resin composition having a haze of 0.1% or more and 10% or less by selecting appropriate molding conditions and composition. A preferable range of the haze is 4% or less, a more preferable range is 2% or less, and an even more preferable range is 1% or less. When the haze exceeds 10%, the transparency targeted by the present embodiment cannot be achieved, and a haze of less than 0.1% is a value that is difficult to achieve in view of the measurement principle. The haze is a value measured in accordance with JIS K 7136:2000.

### <<Base Resin>>

### <Styrenic Resin>

The Applicant will now describe the styrenic resin of the base resin of the resin composition.

The styrenic resin is obtained by polymerizing an aromatic vinyl compound-based monomer, and may be copolymerized with a vinyl monomer as necessary, and may also be rubber-modified by adding a conjugated diene rubbery polymer. As a polymerization method, a known method can be used, for example, bulk polymerization, bulk-suspension two-stage polymerization, solution polymerization, and the like.

As the aromatic vinyl compound-based monomer, known monomers such as styrene, α-methylstyrene, α-methyl p-methylstyrene, o-methylstyrene, m-methylstyrene, p-methylstyrene, vinyltoluene, ethylstyrene, isobutylstyrene, and t-butylstyrene, or bromostyrene and indene, can be used. Among these, styrene is preferable as a main component. In addition, the styrenic resin of the present embodiment can be copolymerized with the above aromatic vinyl compound-based monomers other than styrene, acrylonitrile, (meth)acrylic acid, methyl (meth)acrylate, ethyl (meth)acrylate, propyl (meth)acrylate, butyl (meth)acrylate, or (meth)acrylate esters such as cyclohexyl (meth)acrylate, within a range that does not impair the performance of the styrenic resin of the present embodiment. Furthermore, in the present embodiment, the styrenic resin may be obtained by polymerization with a small amount of a crosslinking agent such as divinylbenzene added thereto.

The conjugated diene rubbery polymer used for rubber modification of the styrenic resin includes polybutadiene; random or block copolymers of styrene-butadiene; polyisoprene; polychloroprene; random, block, or graft copolymers of styrene-isoprene; ethylene-propylene rubber; ethylene-propylene-diene rubber; and the like. Polybutadiene and random, block, or graft copolymers of styrene-butadiene are particularly preferable. These may be partially hydrogenated without any problem.

The content of the conjugated diene rubbery polymer contained in the styrenic resin is preferably 10% by mass or less with respect to 100% by mass of the total amount of the styrenic resin. By setting the content of the rubbery polymer to 10% by mass or less, high fluidity of the styrenic resin can be obtained. In order to have high transparency, the content is more preferably 2% by mass or less. From the viewpoint of impact resistance, the average particle diameter of the conjugated diene rubbery polymer contained in the styrenic resin of the present embodiment is preferably 0.8 µm or more and 3.5 µm or less.

Specific examples of such styrenic resins include polystyrene (GPPS), high-impact polystyrene (HIPS), ABS resin (acrylonitrile-butadiene-styrene copolymer), AS resin (acrylonitrile-styrene copolymer), MS resin (methyl methacrylate-styrene copolymer), ASA resin (acrylonitrile-styrene-acrylic acid ester copolymer), AES resin (acrylonitrile-ethylene propylene-styrene copolymer), MBS resin (methyl methacrylate-butadiene-styrene copolymer), and the like.

The styrenic resin preferably has a weight-average molecular weight of 100,000 or more and 300,000 or less, more preferably 120,000 or more and 250,000 or less, and even more preferably 140,000 or more and 200,000 or less. When the weight-average molecular weight is 100,000 or more and 300,000 or less, a resin having an excellent balance between mechanical properties and fluidity can be obtained, and contamination with gel substances is also small. The weight-average molecular weight is a value obtained by gel permeation chromatography in terms of standard polystyrene.

### <Polycarbonate Resin>

The main component of the polycarbonate resin is an aromatic polycarbonate resin. A typical polycarbonate resin is obtained by reacting a dihydric phenol with a carbonate precursor. Methods for this reaction include an interfacial polycondensation method, a melt transesterification method, a solid-phase transesterification method of a carbonate prepolymer, and a ring-opening polymerization method of a cyclic carbonate compound.

Examples of the dihydric phenol include hydroquinone, resorcinol, 4,4'-biphenol, 1,1-bis(4-hydroxyphenyl)ethane, 2,2-bis(4-hydroxyphenyl)propane (common name: bisphenol A), 2,2-bis(4-hydroxy-3-methylphenyl)propane, 2,2-bis(4-hydroxyphenyl)butane, 1,1-bis(4-hydroxyphenyl)-1-phenylethane, 1,1-bis(4-hydroxyphenyl)cyclohexane, 1,1-bis(4-hydroxyphenyl)-3,3,5-trimethylcyclohexane, 2,2-bis(4-hydroxyphenyl)pentane, 4,4'-(p-phenylenediisopropylidene)diphenol, 4,4'-(m-phenylenediisopropylidene)diphenol, 1,1-bis(4-hydroxyphenyl)-4-isopropylcyclohexane, bis(4-hydroxyphenyl) oxide, bis(4-hydroxyphenyl) sulfide, bis(4-hydroxyphenyl) sulfoxide, bis(4-hydroxyphenyl) sulfone, bis(4-hydroxyphenyl) ketone, bis(4-hydroxyphenyl) ester, 2,2-bis(3,5-dibromo-4-hydroxyphenyl)propane, bis(3,5-dibromo-4-hydroxyphenyl) sulfone, bis(4-hydroxy-3-methylphenyl) sulfide, 9,9-bis(4-hydroxyphenyl)fluorene, and 9,9-bis(4-hydroxy-3-methylphenyl)fluorene, and include isosorbide, isomannide, isoidide, and the like. The cyclic dihydroxy compound includes 3,9-bis(1,1-dimethyl-2-hydroxyethyl)-2,4,8,10-tetraoxaspiro(5.5)undecane (common name: spiroglycol), 3,9-bis(1,1-diethyl-2-hydroxyethyl)-2,4,8,10-tetraoxaspiro(5.5)undecane, 3,9-bis(1,1-dipropyl-2-hydroxyethyl)-2,4,8,10-tetraoxaspiro(5.5)undecane, and the like. Among these, bis(4-hydroxyphenyl)alkane, particularly bisphenol A, is preferable. In addition, a cyclic anhydrosugar alcohol that can be produced from sugars as a biomass-derived raw material, particularly isosorbide, can be inexpensively produced by subjecting D-glucose obtained from starch to a hydrogenation reaction or a dehydration reaction.

In addition, polycarbonate resin obtained by polymerizing at least one of 4,4'-(m-phenylenediisopropylidene)diphenol, 1,1-bis(4-hydroxyphenyl)cyclohexane, 1,1-bis(4-hydroxyphenyl)-3,3,5-trimethylcyclohexane, 9,9-bis(4-hydroxyphenyl)fluorene, and 9,9-bis(4-hydroxy-3-methylphenyl)fluorene as a part or all of the dihydric phenol component is suitable for applications in which requirements for dimensional change due to water absorption and morphological stability are particularly strict. In this case, it is preferable to use these dihydric phenols other than bisphenol A in an amount of 5 mol% or more, particularly 10 mol% or more, with respect to the total dihydric phenol component constituting the polycarbonate.

In addition, as a small amount of a polyhydric phenol component, 1,1,1-tris(4-hydroxyphenyl)ethane, 1,1,1-tris(3,5-dimethyl-4-hydroxyphenyl)ethane, and the like can also be used.

Among the polycarbonate resins, those having a water absorption rate of 0.05% by mass or more and 0.15% by mass or less and those having a glass transition temperature of 120°C or more and 250°C or less, by adjusting a copolymerization composition and the like, are particularly suitable in fields in which morphological stability is required, because the polymer itself has good hydrolysis resistance and also has excellent low warpage after molding.

As the carbonate precursor, carbonyl halides, carbonate esters, dihaloformates, and the like are used. Specific examples include phosgene, diphenyl carbonate, ditolyl carbonate, bis(chlorophenyl) carbonate, m-cresyl carbonate, dinaphthyl carbonate, bis(biphenyl) carbonate, diethyl carbonate, dimethyl carbonate, dibutyl carbonate, dicyclohexyl carbonate, and the like. Diphenyl carbonate is particularly preferable.

In the polycarbonate resin, in addition to the above-described dihydric phenol and carbonate precursor, a catalyst, an end-capping agent, an antioxidant for preventing oxidation of the dihydric phenol, and the like may be used as necessary. The polycarbonate resin is produced by known production methods such as an interfacial polymerization method, a melt transesterification polymerization method, a solid-phase transesterification method of a carbonate prepolymer, and a ring-opening polymerization method of a cyclic carbonate compound.

The polycarbonate resin may be a polyester carbonate obtained by copolymerizing an aromatic or aliphatic (including alicyclic) bifunctional carboxylic acid, a copolycarbonate obtained by copolymerizing a bifunctional alcohol (including alicyclic), or a polyester carbonate obtained by copolymerizing both such a bifunctional carboxylic acid and such a bifunctional alcohol. In addition, a mixture obtained by blending two or more kinds of the obtained polycarbonates may also be used without any problem. As the polycarbonate resin, a resin reclaimed from unnecessary portions generated during production or product processing, or a resin reused after product use, may also be used.

The aliphatic bifunctional carboxylic acid is preferably an α,ω-dicarboxylic acid. Preferred examples of the aliphatic bifunctional carboxylic acid include linear saturated aliphatic dicarboxylic acids such as sebacic acid (decanedioic acid), dodecanedioic acid, tetradecanedioic acid, octadecanedioic acid, and eicosanedioic acid, and alicyclic dicarboxylic acids such as cyclohexanedicarboxylic acid. The bifunctional alcohol is more preferably an alicyclic diol, and examples thereof include cyclohexanedimethanol, cyclohexanediol, tricyclodecanedimethanol, and the like.

Furthermore, as the polycarbonate resin, it is also possible to use a polycarbonate-polyorganosiloxane copolymer obtained by copolymerizing a polyorganosiloxane unit.

The viscosity-average molecular weight of the polycarbonate resin is not limited; however, the viscosity-average molecular weight is preferably in a range of 11,000 or more and 35,000 or less. By setting the viscosity-average molecular weight to 11,000 or more, sufficient strength can be obtained, and by setting the viscosity-average molecular weight to 35,000 or less, molding processability becomes favorable. In this sense, the viscosity-average molecular weight is more preferably in a range of 15,000 or more and 25,000 or less.

### <Acrylic Resin>

The main component of the acrylic resin of the base resin of the present embodiment is a repeating unit derived from methyl methacrylate, and is preferably 85% by mass or more and 99% by mass or less in 100% by mass of the acrylic resin. When the repeating unit derived from methyl methacrylate is 85% by mass or more, the acrylic resin has excellent mechanical properties and heat resistance, and when it is 99% by mass or less, the acrylic resin has excellent thermal stability.

Examples of the alkyl acrylate include methyl acrylate, ethyl acrylate, n-propyl acrylate, iso-propyl acrylate, n-butyl acrylate, iso-butyl acrylate, sec-butyl acrylate, tert-butyl acrylate, n-hexyl acrylate, cyclohexyl acrylate, n-octyl acrylate, 2-ethylhexyl acrylate, and the like. These alkyl acrylates may be used alone or in combination of two or more. Among these alkyl acrylates, methyl acrylate and ethyl acrylate are preferable, and methyl acrylate is more preferable, because the production cost of the acrylic resin can be suppressed.

In addition, the acrylic resin may be copolymerized with repeating units other than methyl methacrylate and alkyl acrylate within a range that does not impair the inherent performance of the acrylic resin. Copolymerizable monomers include (meth)acrylate compounds, (meth)acrylamide compounds, aromatic vinyl compounds, vinyl ether compounds, vinyl carboxylic acid compounds, olefin compounds, and the like. These monomers may be used alone or in combination of two or more.

Examples of the (meth)acrylate compound include ethyl methacrylate, n-propyl methacrylate, iso-propyl methacrylate, n-butyl methacrylate, iso-butyl methacrylate, sec-butyl methacrylate, tert-butyl methacrylate, n-hexyl methacrylate, cyclohexyl methacrylate, n-octyl methacrylate, 2-ethylhexyl methacrylate, phenyl (meth)acrylate, bornyl (meth)acrylate, norbornyl (meth)acrylate, isobornyl (meth)acrylate, adamantyl (meth)acrylate, glycidyl (meth)acrylate, and the like. Examples of the (meth)acrylamide compound include (meth)acrylic acid, (meth)acrylonitrile, (meth)acrylamide, N-dimethyl (meth)acrylamide, N-diethyl (meth)acrylamide, N-butyl (meth)acrylamide, dimethylaminopropyl (meth)acrylamide, N-methylol (meth)acrylamide, N-methoxymethyl (meth)acrylamide, N-butoxymethyl (meth)acrylamide, hydroxyethyl (meth)acrylamide, methylenebis(meth)acrylamide, and the like. Examples of the aromatic vinyl compound include styrene and α-methylstyrene. Examples of the vinyl ether compound include methyl vinyl ether, ethyl vinyl ether, and 2-hydroxyethyl vinyl ether. Examples of the vinyl carboxylic acid compound include vinyl acetate and vinyl butyrate. Examples of the olefin compound include ethylene, propylene, butene, and isobutene.

Examples of the polymerization method of the acrylic resin include bulk polymerization, solution polymerization, suspension polymerization, and emulsion polymerization. Among these polymerization methods, bulk polymerization, solution polymerization, and suspension polymerization are preferable, and bulk polymerization is more preferable, because an emulsifier is unnecessary and the optical properties of the acrylic resin are excellent. Continuous bulk polymerization is even more preferable because it is excellent in productivity and can suppress contamination with foreign substances.

In any polymerization, polymerization is initiated by using a small amount of a polymerization initiator. Examples of the polymerization initiator include organic peroxides and azo compounds. Examples of the organic peroxide include tert-butyl peroxy-3,5,5-trimethylhexanoate, tert-butyl peroxylaurate, tert-butyl peroxyisopropyl monocarbonate, tert-hexyl peroxyisopropyl monocarbonate, tert-butyl peroxyacetate, 1,1-bis(tert-butylperoxy)-3,3,5-trimethylcyclohexane, 1,1-bis(tert-butylperoxy)cyclohexane, tert-butyl peroxy-2-ethylhexanoate, tert-butyl peroxyisobutyrate, tert-hexyl peroxy-2-ethylhexanoate, di-tert-butyl peroxide, di-tert-hexyl peroxide, 2,5-dimethyl-2,5-bis(tert-butylperoxy)hexane, and the like. Examples of the azo compound include 2-(carbamoylazo)-isobutyronitrile, 1,1'-azobis(1-cyclohexanecarbonitrile), 2,2'-azobisisobutyronitrile, 2,2'-azobis(2-methylbutyronitrile), dimethyl 2,2'-azobisisobutyrate, 2,2'-azobis(2,4,4-trimethylpentane), 2,2'-azobis(2-methylpropane), and the like. These polymerization initiators may be used alone or in combination of two or more. Among these polymerization initiators, organic peroxides are preferable, and tert-butyl peroxy-3,5,5-trimethylhexanoate and di-tert-butyl peroxide are more preferable, because the production cost can be suppressed.

The amount of the polymerization initiator to be used is preferably 0.001% by mass or more and 1% by mass or less, and more preferably 0.005% by mass or more and 0.1% by mass or less, with respect to 100% by mass of all monomers, because a desired weight-average molecular weight can be obtained. When the amount of the polymerization initiator is 0.001% by mass or more, the polymerization rate of the monomers is excellent. In addition, when the amount of the polymerization initiator is 1% by mass or less, the production cost can be suppressed.

In addition, it is desirable to use a chain transfer agent in the polymerization reaction system in order to control the molecular weight and the molecular weight distribution. Examples of the chain transfer agent include mercaptan compounds and the like. These chain transfer agents may be used alone or in combination of two or more. Among these chain transfer agents, mercaptan compounds are preferable because the production cost can be suppressed.

Examples of the mercaptan compound include primary, secondary, and tertiary mercaptans having an alkyl group or substituted alkyl group such as n-butyl, iso-butyl, sec-butyl, tert-butyl, n-octyl, n-dodecyl, and sec-dodecyl; aromatic mercaptans such as phenyl mercaptan, thiocresol, and 4-tert-butyl-o-thiocresol; mercaptans having 2 to 18 carbon atoms such as thioglycolic acid and esters thereof, and ethylenethioglycol; and the like. These mercaptan compounds may be used alone or in combination of two or more. Among these mercaptan compounds, n-butyl mercaptan, tert-butyl mercaptan, n-octyl mercaptan, and n-dodecyl mercaptan are preferable, and n-butyl mercaptan and n-octyl mercaptan are more preferable, because the production cost can be suppressed.

The amount of the chain transfer agent to be used is preferably 0.01% by mass or more and 2% by mass or less, and more preferably 0.05% by mass or more and 1% by mass or less, with respect to 100% by mass of all monomers, because a desired weight-average molecular weight can be obtained. When the amount of the chain transfer agent is 0.01% by mass or more, the polymerization stability of the monomers is excellent. In addition, when the amount of the chain transfer agent is 2% by mass or less, the production cost can be suppressed.

The acrylic resin preferably has a weight-average molecular weight of 30,000 or more and 70,000 or less. When the weight-average molecular weight of the acrylic resin is 30,000 or more, the mechanical properties of the acrylic resin are excellent, and when it is 70,000 or less, the moldability of the acrylic resin is excellent. The molecular weight distribution of the acrylic resin is preferably 2.0 or more and 4.0 or less, and more preferably 2.4 or more and 3.6 or less. When the molecular weight distribution of the acrylic resin is 2.0 or more, the moldability of the acrylic resin is excellent, and when it is 4.0 or less, the flow stability of the acrylic resin is excellent.

### <<Acetalized Compound>>

The acetalized compound is obtained by reacting aldehydes with at least a part of hydroxyl groups of a monosaccharide or an oligosaccharide. Here, the monosaccharide refers to a sugar that is not further hydrolyzed. Examples of the monosaccharide include pentoses such as xylose and arabinose, and hexoses such as glucose, mannose, galactose, and fructose, but are not limited thereto. Here, the oligosaccharide refers to a sugar in which monosaccharides are linked by a glycosidic bond in a number of 2 or more and 10 or less. However, a small amount of monosaccharides or polysaccharides in which more than 10 sugars are linked may be included.

As an example of the structure of the acetalized compound of the present embodiment, the structure of a sugar derivative obtained when aldehydes R1-CHO are reacted with xylose, which is a pentose, is shown in (Formula 1). In this way, an acetal structure is obtained in which aldehydes have reacted with the hydroxyl groups at the I-position, the 2-position, and the 3-position and the 4-position of xylose, respectively, thereby obtaining a compound that is relatively bulky but has low rigidity.

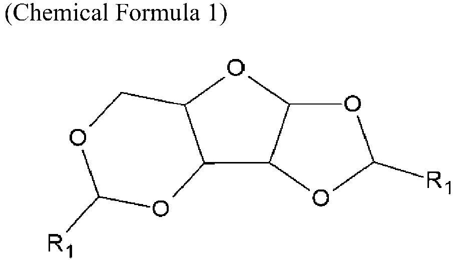

In the case of a hexose, or in the case of an oligosaccharide, aldehydes react with two hydroxyl groups of adjacent sugars, thereby obtaining an acetalized compound. When the sugar is a monosaccharide, the number of acetal structures is one or two, and when the sugar is an oligosaccharide, the number of acetal structures substantially corresponds to the number of monosaccharides.

The acetalized compound also referred to as a sugar derivative has a molecular weight of 200 or more and 1,000 or less, and sufficiently exhibits this characteristic. With an acetal structure having a molecular weight of less than 200, the above-described characteristics of the sugar derivative cannot be obtained, and when the molecular weight exceeds 1,000, the mobility of the sugar derivative (b) decreases. The molecular weight is preferably 300 or more and 600 or less.

The aldehydes have at least two carbon atoms and include at least one aldehyde group (-CHO). Examples thereof include saturated or unsaturated alkyl aldehydes, cyclic aldehydes, glyoxylic acid, and alkyl esters of glyoxylic acid.

In the case of a linear chain, the saturated alkyl aldehyde is a fully saturated compound having a chain length of C2-C20, preferably C2-C14, and is selected from, for example, acetaldehyde, propionaldehyde, butyraldehyde, pentanal (valeraldehyde), hexanal, heptanal, octanal, nonanal, decanal, undecanal, dodecanal, tridecanal, and tetradecanal (myristaldehyde). When having a branched chain, the saturated alkyl aldehyde is a fully saturated compound having a maximum main chain length of C8, particularly C3-C8, and when the branched chain is an alkyl group, examples thereof include isobutyraldehyde, isovaleraldehyde, 2-methylbutyraldehyde, 3,5,5-trimethylhexanal, trimethylacetaldehyde, and 6-methoxy-2,6-dimethyloctanal

The linear unsaturated alkyl aldehyde is a compound having one or more degrees of unsaturation such that the chain length is C3-C20, preferably C3-C8, and is selected from, for example, 2-propenal (acrolein) having one degree of unsaturation, 2-butenal (crotonaldehyde), 3-butenal, trans-2-hexenal, cis-3-hexenal, 2-heptenal, 3-heptenal, 5-heptenal, 2-octenal, 9-decenal, 2,4-pentadienal having two or more degrees of unsaturation, 2,4-hexadienal, 2,4-heptadienal, 3,5-heptadienal, (cis or trans) 2,4-octadienal, and the like. The unsaturated alkyl aldehyde having a branched chain is selected from, for example, 2-methyl-2-butenal (tiglic aldehyde), 2,6-dimethyl-5-heptenal (melonal), 2-methyl-2-pentenal, 2,6,10-trimethyl-9-undecenal (adoksal), and the like.

The unsaturated alkyl aldehyde can have any substituent, and the substituent is selected from citral (lemonal, geranial, neral), citronellal, cinnamaldehyde, α-methylcinnamaldehyde, α-hexylcinnamaldehyde, α-amylcinnamaldehyde (jasmine aldehyde), β-phenylcinnamaldehyde, 2-hydroxycinnamaldehyde, 4-hydroxycinnamaldehyde (coumaraldehyde), 2-methoxycinnamaldehyde, 3,5-dimethoxy-4-hydroxycinnamaldehyde (sinapyl aldehyde), 4-acetoxy-3-methoxycinnamaldehyde, 4-hydroxy-3-methoxycinnamaldehyde (coniferyl aldehyde), 4-chlorocinnamaldehyde, 4-bromocinnamaldehyde, 4-fluorocinnamaldehyde, 2,6-difluorocinnamaldehyde, and the like.

The cyclic aldehyde is a compound of carbaldehydes, that is, a compound in which an aldehyde group is directly bonded to a ring. The ring is a monocyclic ring containing 3 to 8 carbon atoms, and the like. More specifically, the cyclic aldehyde is a non-aromatic ring, an aromatic ring, or an aromatic heterocyclic ring. These may have no substituent or may have a substituent.

Examples of the non-aromatic cyclic aldehyde include cyclopropanecarboxaldehyde, cyclobutanecarboxaldehyde, cyclopentanecarboxaldehyde, cyclohexanecarboxaldehyde, cycloheptanecarboxaldehyde, cyclooctanecarboxaldehyde, and the like. These non-aromatic cyclic aldehydes can have a substituent, and are selected from, for example, perillaldehyde.

Examples of the aromatic cyclic aldehyde include benzaldehyde, 1-naphthaldehyde, 2-naphthaldehyde, and the like, and the substituent is selected from a C1-C8 alkyl group, an ether group, a hydroxy group, and the like, and the substituent may be a plurality of the same or different substituents.

Examples of benzaldehydes substituted with a C1-C8 alkyl group include 2-methylbenzaldehyde, 3-methylbenzaldehyde, and 4-methylbenzaldehyde (respectively, o-, m-, and p-isomers of tolualdehyde), 2,4-dimethylbenzaldehyde, 2,5-dimethylbenzaldehyde, 2,6-dimethylbenzaldehyde, 2,4,6-trimethylbenzaldehyde (mesitaldehyde), 4-ethylbenzaldehyde, 2-ethylbenzaldehyde, biphenyl-2-carboxaldehyde, biphenyl-3-carboxaldehyde, biphenyl-4-carboxaldehyde, 4-tert-butylbenzaldehyde, 2,5-di-tert-butylbenzaldehyde, and 4-isopropylbenzaldehyde (cuminaldehyde).

Examples of benzaldehydes substituted with a hydroxy group include 2-hydroxybenzaldehyde (salicylaldehyde), 3-hydroxybenzaldehyde, 4-hydroxybenzaldehyde, 2,3-dihydroxybenzaldehyde, 2,4-dihydroxybenzaldehyde, 2,5-dihydroxybenzaldehyde (gentisaldehyde), 3,4-dihydroxybenzaldehyde (protocatechualdehyde), 2,3,4-trihydroxybenzaldehyde, 2,4,5-trihydroxybenzaldehyde, 2,4,6-trihydroxybenzaldehyde (phloroglucinol aldehyde), and the like.

Benzaldehydes may have different substituents, and are selected from 2-hydroxy-3-methoxybenzaldehyde, 2-hydroxy-4-methoxybenzaldehyde, 3-hydroxy-4-methoxybenzaldehyde (isovanillin), 4-hydroxy-2-methoxybenzaldehyde, 4-hydroxy-3-methoxybenzaldehyde (vanillin), heliotropin (piperonal), 2,6-dimethoxy-4-hydroxybenzaldehyde, 3,4-dimethoxy-5-hydroxybenzaldehyde, 3,5-dimethoxy-4-hydroxybenzaldehyde (syringaldehyde), 4-ethoxy-3-methoxybenzaldehyde, 3-ethoxy-4-methoxybenzaldehyde, 3-ethoxy-4-hydroxybenzaldehyde (ethylvanillin), 2-ethoxy-3-methoxybenzaldehyde, 2-benzyloxy-3-methoxybenzaldehyde, 3-benzyloxy-4-methoxybenzaldehyde, 4-benzyloxy-3-methoxybenzaldehyde, and the like.

The cyclic aldehyde may be a heteroaromatic aldehyde, and includes furfural, 2-pyridinecarboxaldehyde, 3-pyridinecarboxaldehyde, 4-pyridinecarboxaldehyde, 2-thiazolecarboxaldehyde, pyrrole-2-carboxaldehyde, 3-thiophenecarboxaldehyde, and indole-3-carboxaldehyde. Furthermore, the heteroaromatic aldehyde may have a substituent, and includes 3-methyl-2-thiophenecarboxaldehyde, 6-methylpyridinecarboxaldehyde, N-benzylpyridine-4-carboxaldehyde, 1-methylimidazolecarboxaldehyde, and the like.

The aldehydes of the present embodiment may have two or more aldehyde groups, and may further have a substituent. Specific examples include glyoxal, 2-methylmalondialdehyde, succinaldehyde, glutaraldehyde, bromomalondialdehyde, terephthaldehyde, o-phthaldehyde, m-phthaldehyde, bis(4-formylphenyl) ether, bis(2-formylphenyl) ether, 4,4'-biphenyldicarboxaldehyde, tris(4-formylphenyl)amine, and the like.

The aldehydes are preferably derived from biomass. "Derived from biomass" means that carbon atoms constituting the aldehydes are derived from biomass, and includes not only those extracted and purified from natural products but also those chemically synthesized using natural products as raw materials. In the present embodiment, the biomass-derived carbon atom ratio can be determined from the 14C abundance ratio in total carbon measured in accordance with ASTM D6866. In the present embodiment, the biomass-derived carbon atom ratio of the sugar derivative (b) obtained by this measurement is preferably 50% or more, more preferably 90% by mass or more, and even more preferably 100%.

Examples of such biomass-derived aldehydes include glyoxylic acid, alkyl esters of glyoxylic acid, pentanal (valeraldehyde), hexanal, heptanal, nonanal, decanal, undecanal, dodecanal, tridecanal, tetradecanal (myristaldehyde), isovaleraldehyde, phenylacetaldehyde, 7-hydroxy-3,7-dimethyl-octanal (hydroxycitronellal), D-glyceraldehyde, D-erythrose, trans-2-hexenal, cis-3-hexenal, citral (lemonal, geranial, neral), citronellal, cinnamaldehyde, α-hexylcinnamaldehyde, 4-hydroxycinnamaldehyde (coumaraldehyde), 3,5-dimethoxy-4-hydroxycinnamaldehyde (sinapyl aldehyde), 4-hydroxy-3-methoxycinnamaldehyde (coniferyl aldehyde), perillaldehyde, 4-isopropylbenzaldehyde (cuminaldehyde), 4-methoxybenzaldehyde (anisaldehyde), 3,4,5-trimethoxybenzaldehyde, 4-hydroxybenzaldehyde, 3,4-dihydroxybenzaldehyde (protocatechualdehyde), 2,3,4-trihydroxybenzaldehyde, 2-hydroxy-4-methoxybenzaldehyde, 4-hydroxy-3-methoxybenzaldehyde (vanillin), heliotropin (piperonal), 3,5-dimethoxy-4-hydroxybenzaldehyde (syringaldehyde), furfural, and the like.

Among these aldehydes, anisaldehyde, cinnamaldehyde, furfural, and glyoxylic acid are preferable because they provide excellent transparency, heat resistance, mechanical properties, and moldability as the resin composition of the present embodiment, and among these, cinnamaldehyde is more preferable. Since these four types of aldehydes are widely used as raw materials for industrial products, biomass-derived products thereof are easily obtainable.

The content ratio of the acetalized compound in the resin composition is 3% by mass or more and 30% by mass or less. When the content ratio is less than 3% by mass, a high biomass component ratio cannot be obtained, and when the content ratio exceeds 30% by mass, high transparency cannot be obtained. The content ratio of the acetalized compound in the resin composition is preferably 5% by mass or more and 20% by mass or less, and more preferably 10% by mass or more and 15% by mass or less.

### <<Production Method>>

### <Production Method of Acetalized Compound>

An example of a production method of the acetalized compound of the present embodiment is described below. A raw material sugar is dissolved in a highly polar solvent such as dimethylformamide (DMF) or dimethyl sulfoxide (DMSO), and aldehydes are added in an amount of 2 to 5 equivalents per monosaccharide. As a catalyst, an acid such as hydrochloric acid, sulfuric acid, citric acid, or p-toluenesulfonic acid is added in an appropriate amount. The mixture is heated and stirred under reduced pressure, and thereafter, the mixture is separated with methylene chloride and an aqueous sodium hydroxide solution, and the organic layer is reprecipitated with hexane in an ice bath. Furthermore, crystals are collected by suction filtration, dissolved in methylene chloride or ethyl acetate, and the solution is reprecipitated with methanol, and washing is performed by repeating filtration, cooling, and reprecipitation to obtain an acetalized compound.

### <Production Method of Resin Composition>

The resin composition is produced, for example, by weighing (a) the base resin, (b) the acetalized compound, and, as necessary, a third component at predetermined ratios, and melt-kneading them under heating. It is preferable that each phase of (a) the base resin and (b) the acetalized compound is dispersed to a size sufficiently smaller than the wavelength of visible light so as to improve transparency. Whether dispersion to a size sufficiently smaller than the wavelength of visible light has been achieved can be determined by observation of optical uniformity of a molded article with the naked eye, and by measurement of total light transmittance and haze.

Examples of the method for melt-kneading (a) the base resin and (b) the acetalized compound include a method using a single-screw extruder, a twin-screw kneading extruder, a multi-screw kneading extruder, and the like. The kneading temperature is usually selected as appropriate within a range of 100°C or more and 300°C or less, preferably 150°C or more and 250°C or less, and more preferably 180°C or more and 230°C or less. Prior to melt-kneading, (a) the base resin, (b) the acetalized compound, and, as necessary, the third component may be premixed in a powder or pellet state by a conventional method.

In the resin composition, a third component may be blended as necessary within a range that does not impair the gist of the present embodiment. Examples of such a third component include additives conventionally used for plastics, such as a plasticizer, a dispersant, an antioxidant, an antibacterial agent, a compatibilizer, a lubricant, a processing aid, a mold release agent, a stabilizer, a flame retardant, a transparency improver, a yellowing inhibitor, an ultraviolet absorber, an antistatic agent, an antifogging agent, a pigment, a dye, and a fluorescent dye.

The resin composition may have any form, such as a bulk form, a plate form, a film form, a sheet form, a thread form, a string form, a pellet form, a powder form, a granular form, a particulate form, or any other molded article. From the viewpoint of storage and distribution, the form is preferably a pellet form. The pellet shape may be any of a cylindrical shape, an elliptical cylindrical shape, a flat plate shape, a spherical shape, an ellipsoidal shape, and the like, and examples thereof include a cylindrical shape having a diameter of 0.5 mm or more and 5 mm or less and a length of 0.5 mm or more and 10 mm or less.

### <Production Method of Molded Article>

The resin composition having excellent transparency of the present embodiment is preferably used as a molded article including the resin composition at least partially. Since the resin composition of the present embodiment is thermoplastic, the resin composition can be processed into a molded article having a desired shape by heating to melt or fluidize and soften the resin composition, charging the resin composition into a mold, compressing the resin composition, or extruding the resin composition. The melting and molding temperature is usually 200°C or more and 230°C or less, but is not limited thereto. As an index of moldability, an MFR value was used. The MFR in Table 1 was measured in accordance with JIS K 7210-1:2014.

Examples of the molding method for the molded article include injection molding, extrusion molding, casting molding, calender molding, slush molding, blow molding, vacuum molding, powder molding, foam molding, extrusion lamination molding, T-die molding, air-cooled inflation molding, water-cooled inflation molding, microwave molding which is a mold is heated by radiation, and the like. In some cases, the molded article may be formed by deforming (molding) using its own weight without using a mold, or by applying an external force using a tool. The molded article can be molded into various shapes such as a film, a sheet, a thin plate, a thick plate, a corrugated plate, a thread, a filament, a rod, a pipe, a column, a modeled object, and an artwork.

In addition, since the resin composition of the present embodiment has excellent heat resistance, mechanical properties, and moldability, molded articles thereof can be widely used for windows such as windows that partition indoor and outdoor spaces or rooms of transparent buildings and vehicles, partition plates, water tanks, and carport roof panels; lighting apparatuses, flat panel displays, mobile phones, automobile headlight covers, signs, showcases, and covers such as watch covers; lenses such as illumination lenses, optical lenses, eyeglasses, and sunglasses; sheets such as blisters and carrier tapes; films such as agricultural product packaging and window films for paper boxes and envelopes; and other daily necessities such as ceiling materials, ballpoint pens, and cups, and ornaments.

### Examples

### <Synthesis of Acetalized Compound>

Synthesis of the acetalized compounds of Examples 1 to 10 will be described. A predetermined amount of a raw material sugar (xylose or xylooligosaccharide) was dissolved in a dimethylformamide (DMF) solution, and aldehydes were added in an amount of 2 to 5 equivalents per monosaccharide. At that time, p-toluenesulfonic acid was added in an appropriate amount to the DMF solution as a catalyst. The solution was heated and stirred under reduced pressure at 40°C to 60°C for 4 hours or more, and thereafter, the mixture was separated with methylene chloride and a 2 wt% aqueous sodium hydroxide solution, and the organic layer was reprecipitated with hexane.

After crystallization, pale yellow crystals were collected by filtration, and the crystals were dissolved in methylene chloride or ethyl acetate, and the solution was reprecipitated with methanol. Thereafter, the crystals were washed by repeating filtration, dissolution, and reprecipitation three times. The crystals were dried in a vacuum dryer at 60°C for 12 hours or more to obtain a white aldehyde compound as a sugar derivative.

### <Production of Resin Composition>

A sugar derivative was mixed with a predetermined transparent resin so as to achieve a predetermined mass ratio, and the mixture was sufficiently mixed using a blender or the like. Then, the mixture was kneaded at 180°C to 230°C using a twin-screw extruder (KZW25TW-45MG-NH300 manufactured by TECHNOVEL), and pellets of the resin composition were obtained by cutting filaments with a pelletizer. The pellets of Examples 1 to 10 and Comparative Examples 1 to 2 had a cylindrical shape with a diameter of 2 to 5 mm and a length of 5 to 7 mm.

### <Preparation of Test Specimens for Physical Property Evaluation>

The pellets of the resin composition were molded using a small injection molding machine (C Mobile-0813 manufactured by Epson Tech Form Co., Ltd.) at 210°C to prepare and evaluate a flat plate of 40 mm × 40 mm × 1 mm, a strip test specimen of 80 mm × 10 mm × t2 mm, and a 5A-size dumbbell. The mold surface of the test specimen is preferably mirror-polished as appropriate using abrasive paper, fixed abrasives, free abrasives, and the like. Cleaning of the injection molding machine was sufficiently performed.

### <Measurement and Evaluation Methods>

### (1) Molecular Weight of Acetalized Compound

When the raw material sugar was xylose, the molecular weight of the sugar derivative was defined as the molecular weight of a structure in which aldehydes reacted with hydroxyl groups of xylose. When the raw material sugar was xylooligosaccharide, the absolute molecular weight was determined by GPC-MALS-RI using the apparatus and the like described below.

System: ACQUITY Arc Empower3 (manufactured by Nihon Waters K.K.)
Detectors: (MALS) DAWN (manufactured by Wyatt Technology Corporation)
(RI) 2414 RI (manufactured by Nihon Waters K.K.)
Columns: KF-804L (8 mm I.D. × 30 cm) × 2 (manufactured by Shodex)
Eluent: THF (tetrahydrofuran)
Measurement temperature: 40°C
Flow rate: 0.7 mL/min
Sample concentration: 1 mg/mL
Injection volume: 50 µL

### (2) Total Light Transmittance

In accordance with JIS K 7375:2008, the test specimen thickness was set to 1 mm, and measurement was performed using a haze meter manufactured by Nippon Denshoku Industries Co., Ltd.

### (3) Haze

In accordance with JIS K 7136:2000, the test specimen thickness was set to 1 mm, and measurement was performed using a haze meter manufactured by Nippon Denshoku Industries Co., Ltd.

### (4) Tensile Test

In accordance with JIS K 7161-2:2014, using the following test specimen and measurement apparatus, a tensile modulus (maximum value, MPa) and a strain at break (elongation at break, %) were measured.
Test specimen: Type 5A (small test specimen)
Measurement apparatus: Universal testing machine Model 5966 manufactured by Instron Corporation
Test speed: 2.5 mm/min
Distance between chucks: 50 mm

### (5) Flexural Modulus

In accordance with JIS K 7171:2016, using the following test specimen and measurement apparatus, a flexural modulus was measured.
Test specimen: 80 mm × 10 mm × t2 mm
Measurement apparatus: Universal testing machine Model 5966 manufactured by Instron Corporation
Test speed: 1 mm/min
Span length: 32 mm

### (6) Deflection Temperature Under Load

In accordance with JIS K 7191-2:2015, using the following test specimen and measurement apparatus, a deflection temperature under load was measured.
Test specimen: 80 mm × 10 mm × t2 mm
Measurement apparatus: Heat Distortion Tester 148-HD-PC manufactured by Yasuda Seiki Seisakusho, Ltd.
Flexural stress: 1.80 MPa (Method A)
Span length: 64 mm
Heating rate: 120°C/hr
Heat medium: silicone oil

### (7) MFR (Melt Flow Rate)

In accordance with JIS K 7210-1:2014, MFR was measured using the following measurement apparatus.
Measurement apparatus: Melt Indexer LMFI5500 (manufactured by Nippon Dynisco Co., Ltd.)
Temperature and load: 200°C, 5 kg (PS), 230°C, 2.16 kg (PMMA, PC)

The physical properties of the compositions corresponding to Examples 1 to 10 are shown in Table 1. In addition, the physical properties corresponding to Comparative Examples 1 to 4 and the physical properties of a styrenic resin (PSJ-Polystyrene GPPS (grade name: HF77) manufactured by PS Japan Corporation), a polycarbonate resin (Panlite^{®} (registered trademark) (grade name: AD-5503) manufactured by Teijin Limited), and an acrylic resin (Acrypet^{®} (registered trademark) (grade name: TF-9) manufactured by Mitsubishi Chemical Corporation) are shown in Table 2.

**[Table 1]**

| **Item** | **Unit** | **Example 1** | **Example 2** | **Example 3** | **Example 4** | **Example 5** | **Example 6** |
|---|---|---|---|---|---|---|---|
| Base Resin | | PS | PS | PS | PS | PS | PS |
| Content Ratio of Base Resin | % | 90 | 90 | 90 | 90 | 90 | 90 |
| Raw Material Sugar | | Xylose | Xylose | Xylose | Xylose | Xylose | Xylose |
| Aldehydes | | Cinnamaldehyde | Acrolein | Furfural | Glyoxyllc Acid | Anisaldehyde | 3,4,5-Trimethoxybenz aldehyde |
| Molecular Weight of Acetalized Compound | | 378 | 226 | 306 | 262 | 386 | 506 |
| Content Ratio of Acetalized Compound | % | 10 | 10 | 10 | 10 | 10 | 10 |
| Content Ratio of Unreacted Sugar | % | - | - | - | - | - | - |
| Total Light Transmittance | % | 89.5 | 69.9 | 89.7 | 89.5 | 89.4 | 89.3 |
| Haze | % | 0.6 | 0.4 | 0.5 | 0.5 | 0.4 | 0.4 |
| Tensile Modulus | MPa | 4060 | 4030 | 4050 | 4000 | 4010 | 3990 |
| Strain al Break | % | 1.7 | 1.8 | 1.7 | 1.8 | 1.7 | 1.6 |
| Flexural Modulus | Mpa | 2980 | 2960 | 2990 | 2890 | 2910 | 2930 |
| Deflection Temperature Lindar Load | °C | 70 | 69 | 70 | 68 | 70 | *71* |
| MFR | g/10min | 31.5 | 32.1 | 33.3 | 34.3 | 33.5 | 32.5 |

| **Item** | **Unit** | **Example 7** | **Example 8** | **Example 9** | **Example 10** | **Example 11** | **Example 12** |
|---|---|---|---|---|---|---|---|
| Base Resin | | PS | PMMA | PC | PS | PS | PS |
| Content Ratio of Base Resin | % | 90 | 90 | 90 | 95 | 85 | 70 |
| Raw Material Sugar | | Xylooligosacchar ide | Xylose | Xylose | Xylose | Xylose | Xylose |
| Aldehydes | | Cinnamaldehyde | Cinnamaldehyde | Cinnamaldehyde | Cinnamaldehyde | Cinnamaldehyde | Cinnamaldehyde |
| Molecular Weight of Acetalized Compound | | 897 | 378 | 378 | 378 | 378 | 378 |
| Contant Ratio of Acetalized Compound | % | 10 | 10 | 10 | 5 | 15 | 30 |
| Content Ratio of Unreacted Sugar | % | - | - | - | - | - | - |
| Total Light Transmittance | % | 85.7 | 90.2 | 86.6 | 89.7 | 88.9 | 82.5 |
| Haze | % | 0.9 | 0.5 | 0.6 | 0.8 | 0.9 | 3.5 |
| Tensile Modulus | MPa | 4010 | 3910 | 2910 | 3950 | 3710 | 3460 |
| Slraln all Break | % | 1.4 | 16 | 12 | 18 | 14 | 1.1 |
| Flexural Madulus | Mpa | 2830 | 2700 | 2010 | 3100 | 2750 | 2530 |
| Deflection Temperature Under Load | °C | 73 | 68 | 107 | 73 | 66 | 62 |
| MFR | g/10min | 27.8 | 35.2 | 18.3 | 25.8 | 45.2 | 118.2 |

**[Table 2]**

| **Item** | **Unit** | **Comparative Example 1** | **Comparative Example 2** | **Comparative Example 3** | **Comparative Example 4*** | **HF77** | **TF-9** | **AD-5503** |
|---|---|---|---|---|---|---|---|---|
| Base Resin | | PS | PS | PS | PS | PS | PMMA | PC |
| Content Ratio of Base Resin | % | 90 | 65 | 90 | 90 | 100 | 100 | 100 |
| Raw Material Sugar | | Xylooligosacchar ide | Xylose | Xylooligosacchar ide | Xylose | - | - | - |
| Aldehydes | | 3,4,5-Trimethoxybenz aldehyde | Cinnamaldehyde | - | - | - | - | - |
| Molecular Weight of Acetalized Compound | | 1089 | 378 | - | - | - | - | - |
| Content Ratio of Acetalized Compound | % | 10 | 35 | - | - | - | - | - |
| Content Ratio of Unreacted Sugar | % | - | - | 10 | 10 | - | - | - |
| Total Light Transmittance | % | 69.8 | 75.3 | 43.6 | - | 90.0 | 92.2 | 89.7 |
| Haze | % | 17.8 | 10.3 | 99.2 | - | 0.9 | 0.6 | 0.3 |
| Tensile Modulus | MPa | 3790 | 3280 | 3930 | - | 3760 | 3950 | 2890 |
| Strain at Break | % | 0.7 | 0.9 | 1,3 | - | 2.1 | 1.8 | 58 |
| Flexural Modulus | Mpa | 2450 | 2390 | 2630 | - | 3170 | 2730 | 2120 |
| Deflection Temperature Under Load | °C | 72 | 59 | 77 | - | 78 | 74 | 120 |
| MFR | g/10min | 29.3 | 135.6 | 46.1 | 31.5 | 8.5 | 10.7 | 6.9 |

| | | | | | | | | |
|---|---|---|---|---|---|---|---|---|
| *Comparative Example 4 could be kneaded, but could not be injection-molded. | | | | | | | | |

### (Example 1)

In the resin composition of Example 1, the base resin was a styrenic resin and the ratio thereof in the composition was 90% by mass, and the acetalized compound was obtained by reacting xylose as the raw material sugar with cinnamaldehyde (derived from biomass). The molecular weight of the acetalized compound was 378.

As shown in Table 1, as compared with the physical properties of the styrenic resin (HF77) (Table 2), it can be seen that the physical properties were almost the same (however, the MFR was increased).

### (Example 2)

In the resin composition of Example 2, the base resin was a styrenic resin and the ratio thereof in the composition was 90% by mass, and the acetalized compound was obtained by reacting xylose as the raw material sugar with acrolein (non-biomass). The molecular weight of the acetalized compound was 226.

As compared with the physical properties of the styrenic resin (HF77), the physical properties were almost the same (however, the MFR was increased), and it can be seen that the physical properties were similar to those of the resin composition of Example 1.

### (Example 3)

In the resin composition of Example 3, the base resin was a styrenic resin and the ratio thereof in the composition was 90% by mass, and the acetalized compound was obtained by reacting xylose as the raw material sugar with furfural (derived from biomass). The molecular weight of the acetalized compound was 306.

As compared with the physical properties of the styrenic resin (HF77), the physical properties were almost the same (however, the MFR was increased), and it can be seen that the physical properties were similar to those of the resin compositions of Examples 1 to 2.

### (Example 4)

In the resin composition of Example 4, the base resin was a styrenic resin and the ratio thereof in the composition was 90% by mass, and the acetalized compound was obtained by reacting xylose as the raw material sugar with glyoxylic acid (derived from biomass). The molecular weight of the acetalized compound was 262.

As compared with the physical properties of the styrenic resin (HF77), the physical properties were almost the same (however, the MFR was increased), and it can be seen that the physical properties were similar to those of the resin compositions of Examples 1 to 3.

### (Example 5)

In the resin composition of Example 5, the base resin was a styrenic resin and the ratio thereof in the composition was 90% by mass, and the acetalized compound was obtained by reacting xylose as the raw material sugar with anisaldehyde (derived from biomass). The molecular weight of the acetalized compound was 386.

As compared with the physical properties of the styrenic resin (HF77), the physical properties were almost the same (however, the MFR was increased), and it can be seen that the physical properties were similar to those of the resin compositions of Examples 1 to 4.

### (Example 6)

In the resin composition of Example 6, the base resin was a styrenic resin and the ratio thereof in the composition was 90% by mass, and the acetalized compound was obtained by reacting xylose as the raw material sugar with 3,4,5-trimethoxybenzaldehyde (derived from biomass). The molecular weight of the acetalized compound was 506.

As compared with the physical properties of the styrenic resin (HF77), the physical properties were almost the same (however, the MFR was increased), and it can be seen that the physical properties were similar to those of the resin compositions of Examples 1 to 5.

### (Example 7)

In the resin composition of Example 7, the base resin was a styrenic resin and the ratio thereof in the composition was 90% by mass, and the acetalized compound was obtained by reacting xylooligosaccharide as the raw material sugar with cinnamaldehyde. The molecular weight of the acetalized compound was 897.

As compared with the physical properties of the styrenic resin (HF77), the physical properties were almost the same (however, the MFR was increased), and it can be seen that the physical properties were also almost similar even as compared with the physical properties of the resin composition of Example 1 in which the raw material sugar was different.

### (Example 8)

In the resin composition of Example 8, the base resin was an acrylic resin (TF-9) and the ratio thereof in the composition was 90% by mass, and the acetalized compound was obtained by reacting xylose as the raw material sugar with cinnamaldehyde. The molecular weight of the acetalized compound was 378.

It can be seen that the physical properties of the resin composition of Example 8 were almost the same as those of the acrylic resin (TF-9) (however, the MFR was increased).

### (Example 9)

In the resin composition of Example 9, the base resin was a polycarbonate resin (AD-5503) and the ratio thereof in the composition was 90% by mass, and the acetalized compound was obtained by reacting xylose as the raw material sugar with cinnamaldehyde. The molecular weight of the acetalized compound was 378.

It can be seen that the physical properties of the resin composition of Example 9 were almost the same as those of the polycarbonate resin (AD-5503) (however, the MFR was increased and the strain at break was decreased).

### (Example 10, Example 11, Example 12, Comparative Example 2)

Examples 10, 11, and 12 and Comparative Example 2 use the same base resin and acetalized compound as those of Example 1, but are examples in which the ratio of the base resin in the resin composition differs and is 95% by mass, 85% by mass, 70% by mass, and 65% by mass, respectively.

It can be seen that the physical properties of the resin compositions of Examples 10 and 11 were almost the same as those of the styrenic resin (HF77) (however, the MFR was increased), and were almost unchanged from the physical properties of the resin composition of Example 1.

On the other hand, in the physical properties of the resin composition of Example 12, the haze was slightly worsened to 3.5%, and the MFR was significantly increased. In the physical properties of the resin composition of Comparative Example 2, the total light transmittance was 75.3%, which is below the target total light transmittance of 80%, and furthermore, the haze was 10.3%, which exceeds 4%, which is a preferable value of haze. Therefore, the base resin preferably has a content ratio of 70% by mass or more in the resin composition, and more preferably 85% by mass or more.

### (Comparative Example 1)

In the resin composition of Comparative Example 1, the base resin was a styrenic resin and the ratio thereof in the composition was 90% by mass, and the acetalized compound was obtained by reacting xylooligosaccharide as the raw material sugar with 3,4,5-trimethoxybenzaldehyde (derived from biomass). The molecular weight of the acetalized compound was 1,089.

In the physical properties of the resin composition of Comparative Example 1, the total light transmittance was 69.8%, which is below the target total light transmittance of 80%, and furthermore, the haze was 17.8%, which exceeds 4%, which is a preferable value of haze. As compared with Example 4 or Example 5, it can be inferred that when the molecular weight of the acetalized compound increases, the total light transmittance and the haze deteriorate. In consideration of the molecular weights of the acetalized compounds of Examples 1 to 10, it is considered preferable that the molecular weight of the acetalized compound is 200 or more and 1,000 or less.

### (Comparative Example 3, Comparative Example 4)

The resin compositions of Comparative Examples 3 and 4 are resin compositions in which the base resin was a styrenic resin and the ratio thereof in the composition was 90% by mass, and 10% by mass of xylooligosaccharide, and 10% by mass of xylose, which is a monosaccharide itself, were kneaded therein, respectively. That is, the resin compositions of Comparative Examples 3 and 4 do not use an acetalized compound.

In the physical properties of the resin composition of Comparative Example 3, the total light transmittance was 43.6%, which is below the target total light transmittance of 80%, and furthermore, the haze was 99.2%, which greatly exceeds 4%, which is a preferable value of haze. In addition, in the resin composition of Comparative Example 4, since injection molding was impossible, test specimens could not be produced. Therefore, the physical property values of Comparative Example 4 are not described.

## Claims

1. A resin composition having excellent transparency, comprising:
a base resin containing at least one of a styrenic resin, a polycarbonate resin, and an acrylic resin; and
an acetalized compound in which at least a part of hydroxyl groups of a monosaccharide or an oligosaccharide has reacted with aldehydes,
wherein the base resin has a content ratio in the resin composition of 70% by mass or more and 97% by mass or less, and
the acetalized compound has a molecular weight of 200 or more and 1,000 or less.

2. The resin composition having excellent transparency according to claim 1, wherein the base resin has a content ratio in the resin composition of 85% by mass or more and 95% by mass or less.

3. The resin composition having excellent transparency according to claim 1, wherein the aldehydes are derived from biomass.

4. The resin composition having excellent transparency according to claim 3, wherein the aldehydes include at least one selected from anisaldehyde, cinnamaldehyde, furfural, and glyoxylic acid.

5. The resin composition having excellent transparency according to claim 4, wherein the aldehydes are cinnamaldehyde.

6. A molded article comprising the resin composition having excellent transparency according to any one of claims 1 to 5.
